(19) Europäisches Patentamt
European Patent Office
Office européen des brevets

(11) Numéro de publication: 0 192 288
B1

(12) FASCICULE DE BREVET EUROPÉEN

(45) Date de publication du fascicule du brevet: 13.09.89

(51) Int. Cl.⁴: **C 07 J 41/00**, C 07 J 5/00, C 07 J 13/00

(21) Numéro de dépôt: 86200123.7

(22) Date de dépôt: 15.02.83

(54) **Nouveaux dérivés stéroides portant en 17 un radical nitrométhylène, Leur application à la préparation de produits biologiquement actifs.**

(30) Priorité: 18.02.82 FR 8202681

(43) Date de publication de la demande: 27.08.86 Bulletin 86/35

(45) Mention de la délivrance du brevet: 13.09.89 Bulletin 89/37

(84) Etats contractants désignés: AT CH DE FR GB IT LI NL SE

(56) Documents cité:
EP-A-0 023 856
DE-A-2 148 631
GB-A-2 079 756

THE JOURNAL OF ORGANIC CHEMISTRY, vol. 35, no. 5, mai 1970, pages 1389-1392; G.R. PETTIT et al.: "Bufadienolides. 5. Synthesis of cardenolides"
JOURNAL OF THE CHEMICAL SOCIETY, CHEMICAL COMMUNICATIONS, no. 15, 1er août 1981, pages 774-775, Londres, GB; D.H.R. BARTON et al.: "Efficient synthesis of the corticosteroid side-chain from 17-ketones"
JOURNAL OF THE CHEMICAL SOCIETY, CHEMICAL COMMUNICATIONS, vol. 10, mai 1982, pages 551-552, Londres, GB; D.H.R. BARTON et al.: "A simple construction of the hydroxy-ketone side chain of corticosteroids from 17-oxo-steroids via nitro-olefins"

(73) Titulaire: ROUSSEL- UCLAF, 35, boulevard des Invalides, F-75007 Paris (FR)

(72) Inventeur: Barton, Derek Harold Richard Bâtiment Résidentiel, du C.N.R.S. Route de Château Fort, F-91190 Gif sur Yvette (FR)
Inventeur: Motherwell, William Branks, 204 Waverley Avenue, Twickhenham Middlesex TW2 6DL (GB)
Inventeur: Zard Zard, Samir, Appt. 96 4, Résidence des Fonds Fanettes, F-91190 Gif Sur Yvette (FR)

(74) Mandataire: Tonnellier, Marie- José, Département des Brevets ROUSSEL UCLAF B.P. no 9, F-93230 Romainville (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 192 288 B1

LIBER, STOCKHOLM 1989

**Description**

La présente invention concerne de nouveaux dérivés stéroïdes portant en 17, un radical nitrométhylène, leur application à la préparation de produits biologiquement actifs.

L'invention a pour objet, à titre de composés nouveaux, les composés de formule $I_A$:

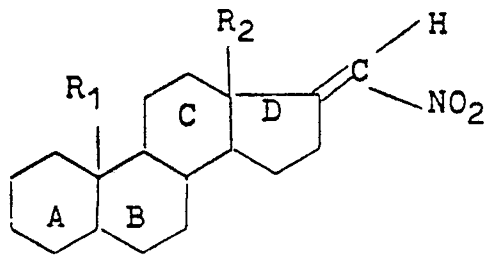

$(I_A)$

dans laquelle $R_1$ représente un atome d'hydrogène, un radical alkyle renfermant de 1 à 4 atomes de carbone éventuellement substitué par une fonction oxygénée ou azotée ou par un atome d'halogène où $R_1$ représente un radical alkényle ou alkynyle renfermant de 2 à 4 atomes de carbone, $R_2$ représente un radical alkyle renfermant de 1 à 4 atomes de carbone, les noyaux A, B, C et D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone ou par un ou plusieurs radicaux alkényle et alkynyle renfermant de 2 à 4 atomes de carbone.

Dans les composés de formules (I), $R_1$, $R_2$, A, B, C et D ont de préférence les significations suivantes:

Lorsque $R_1$ représente un radical alkyle, il s'agit de préférence du radical méthyle ou éthyle.

Lorsque $R_1$ représente un radical alkyle substitué par une fonction oxygénée, il s'agit de préférence du radical hydroxy méthyle ou hydroxy éthyle, du radical formyle ou du radical acétyle.

Lorsque $R_1$ représente un radical alkyle substitué par une fonction azotée, il s'agit de préférence du radical cyano ou du radical amino méthyle ou amino éthyle.

Lorsque $R_1$ représente un radical alkyle substitué par un halogène, il s'agit de préférence d'un radical $-CH_2Hal$, dans lequel Hal représente un atome d'halogène, comme, par exemple, un atome de chlore, de fluor ou de brome.

Lorsque $R_1$ représente un radical alkényle, il s'agit de préférence du radical vinyle ou allyle.

Lorsque $R_1$ représente un radical alkynyle, il s'agit de préférence du radical éthynyle.

$R_2$ représente de préférence un radical méthyle ou éthyle.

Lorsque les noyaux A, B, C et D portent une ou plusieurs doubles liaisons, il s'agit de préférence de doubles liaisons en 1(2), 3(4), 4(5) ou 9(11) ou d'un système de doubles liaisons conjugées en 3(4) et 5(6) ou en 4(5) et 6(7) ou en 1(2) et 4(5) ou d'un système aromatique de trois doubles liaisons 1, 3, 5 ou d'un système de trois doubles liaisons 1(2), 4(5), 6(7).

Lorsque les cycles A, B, C et D sont substitués par une ou plusieurs fonctions hydroxyles, il s'agit de préférence d'une fonction hydroxyle en 3 ou en 11β.

Lorsque les cycles A, B, C et D sont substitués par une ou plusieurs fonctions cétones, il s'agit de préférence d'une fonction cétone en 3 ou en 11.

Lorsque les cycles A, B, C et D sont substitués par un ou plusieurs atomes d'halogènes, il s'agit de préférence d'un atome de fluor, de chlore ou de brome en position 6 ou 9α par exemple.

Lorsque les cycles A, B, C et D sont substitués par un ou plusieurs radicaux alkyles, il s'agit de préférence du radical méthyle ou éthyle en 2, 6, 7 en 16α ou en 16β.

Lorsque les cycles A, B, C et D sont substitués par un ou plusieurs radicaux alkyloxy, il s'agit de préférence d'un radical méthoxy ou éthoxy en 3 ou 11β.

Lorsque les cycles A, B, C et D sont substitués par un ou plusieurs radicaux alkényles, il s'agit de préférence du radical vinyle ou allyle en position 11β par exemple.

Lorsque les cycles A, B, C et D sont substitués par un ou plusieurs radicaux alkynyles, il s'agit de préférence du radical éthynyle en position 11β par exemple.

L'invention a notamment pour objet les composés de formule I dans laquelle $R_2$ représente un radical méthyle et $R_1$ un atome d'hydrogène ou un radical méthyle.

L'invention a tout spécialement pour objet les composés de formule $I'_A$:

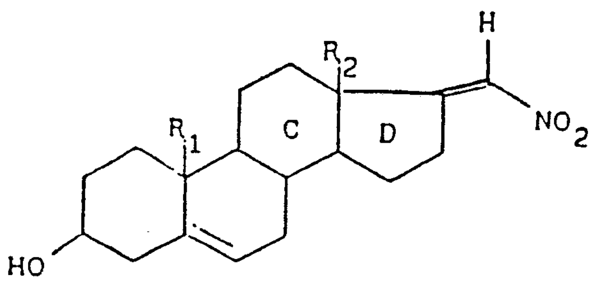

(I'$_A$)

dans laquelle R, $R_2$, C et D conservent leur signification précédente, et notamment les composés de formule I''$_A$:

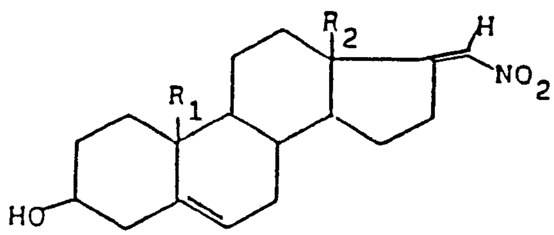

(I''$_A$)

comme par exemple le 3β-hydroxy 17-(nitrométhyl-ène) androst 5-ène.

Les composés de l'invention sont préparés en faisant réagir un composé de formule $II_A$:

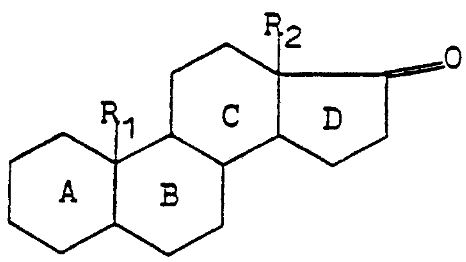

($II_A$)

avec le nitrométhan, utilisé en présence d'un catalyseur basique bifonctionnel, comme il est indiqué dans la demande de brevet européen 0 087 359. Comme catalyseur basique bifonctionnel, on peut utiliser une diamine, par exemple l'éthylène diamine, la triméthylène diamine et la tétraméthylène diamine.

Les produits de formule $I_A$ sont d'un très grand intérêt industriel. Ils sont en effet préparés directement avec d'excellents rendements à partir des composés 17-cétoniques correspondants par un procédé simple et économique: ils peuvent être facilement transformés en composés 21-acétoxy 20-oxo corticostéroïdes portant ou non une double liaison en 16(17), sans qu'il soit nécessaire d'isoler les produits intermédiaires obtenus.

Les composés de formule $I_A$ peuvent servir notamment à préparer les produits décrits dans la demande de brevet allemand 2 521 231, dans les brevets français 1 058 850 et 1 021 728 dans le brevet américain 3 445 490.

Les produits de formule $I_A$ peuvent donc être utilisés dans la préparation de produits présentant un très grand intérêt comme médicaments, comme, par exemple, la triamcinolone et ses dérivés.

L'invention a également pour objet l'application des composés de formule $I_A$ tels que définis précédemment, caractérisée en ce que l'on soumet un composé de formule $I_A$ à l'action d'un aldéhyde RCHO dans lequel R représente un atome d'hydrogène ou un radical alkyle renfermant de 1 à 18 atomes de carbone, pour obtenir un composé de formule (III):

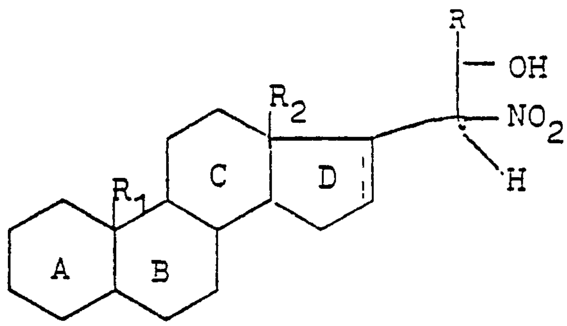

(III)

dans laquelle

A, B, C, D, R, $R_1$ et $R_2$ sont définis comme précédemment, que l'on soumet à l'action d'un agent d'estérification pour obtenir le composé de formule (IV):

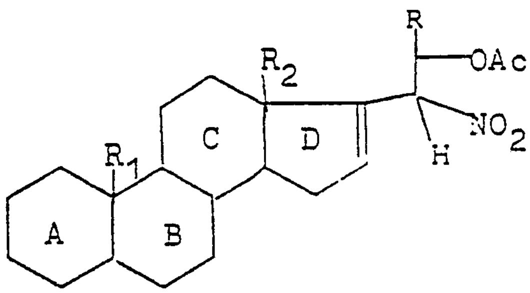

(IV)

dans laquelle

Ac représente le reste d'un radical acyle renfermant de 1 à 18 atomes de carbone que l'on soumet à l'action d'un agent de réduction du groupement nitro pour obtenir le composé de formule (V):

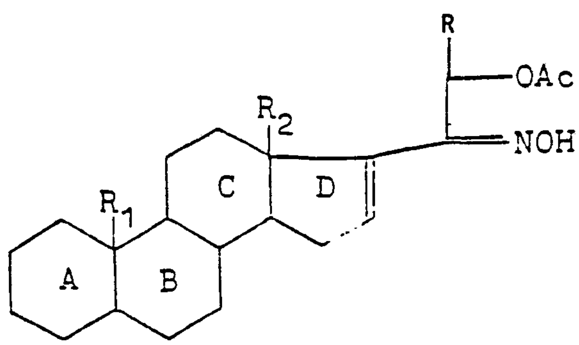

(V)

que l'on soumet à un agent de clivage de la fonction oxime, pour obtenir le composé de formule (VI):

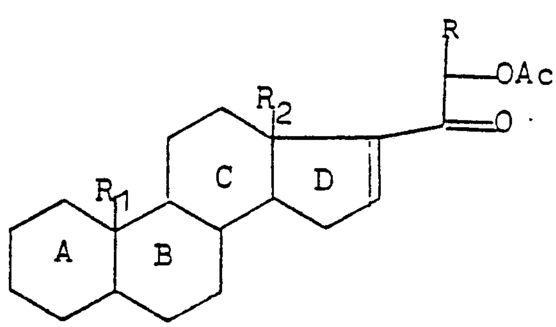

(VI)

Dans un mode de réalisation préféré,

- l'aldéhyde utilisé est le formaldéhyde ou l'acétaldéhyde;
- l'agent d'estérification est l'acide acétique, l'anhydride acétique, le chlorure d'acétyle ou tout autre anhydride ou chlorure d'acide utilisé avec ou sans catalyseur;

- l'agent de réduction du groupement nitro est le chlorure chromeux ou le trichlorure de titane;
- l'agent de clivage du groupement oxime est une solution aqueuse de trichlorure de titane ou l'acide acétique en présence de nitrate alcalin ou un acide minéral aqueux en présence d'un aldéhyde.

On peut signaler également une variante de l'application qui vient d'être décrite, caractérisée en ce que l'on soumet un composé de formule ($I_A$):

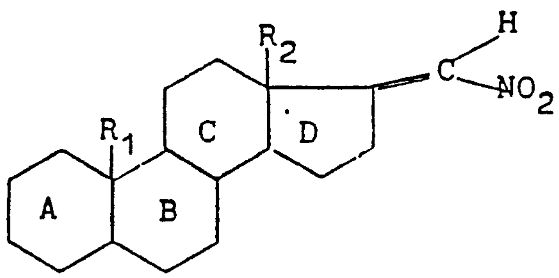

($I_A$)

dans laquelle

A, B, C, D, $R_1$ et $R_2$ sont définis comme précédemment d'abord, à l'action d'un agent de réduction de la double liaison 17(20), pour obtenir le composé de formule (VII):

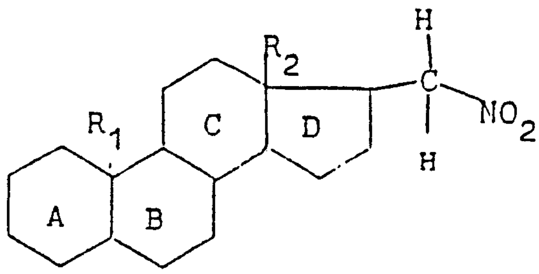

(VII)

que l'on soumet ensuite à la suite des réactions indiquées ci-dessus, c'est-à-dire d'abord à l'action d'un aldéhyde RCHO tel que défini précédemment, pour obtenir le composé de formule (III'):

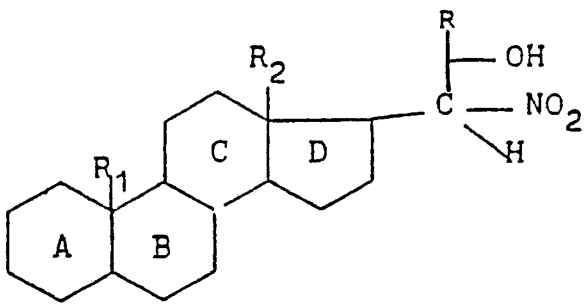

(III')

dans laquelle A, B, C, D, R, $R_1$ et $R_2$ sont définis comme précédemment que l'on soumet à l'action d'un agent d'estérification, pour obtenir le composé de formule (IV'):

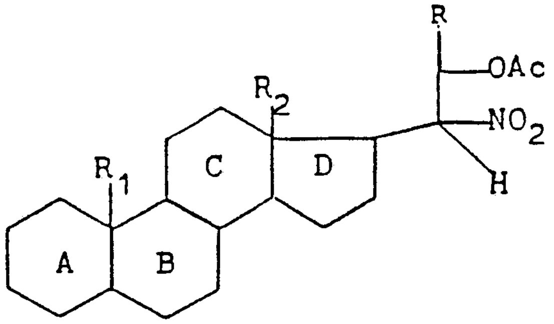

(IV')

dans laquelle

Ac représente le reste d'un radical acyle renfermant de 1 à 18 atomes de carbone, que l'on soumet à l'action d'un agent de réduction du groupement nitro pour obtenir le composé de formule (V'):

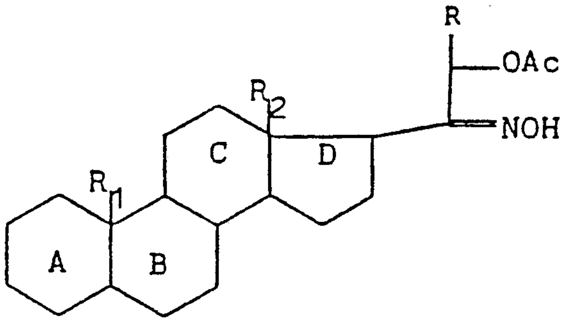

(V')

que l'on soumet à l'action d'un agent de clivage de la fonction oxime, pour obtenir le composé de formule (VI') correspondant:

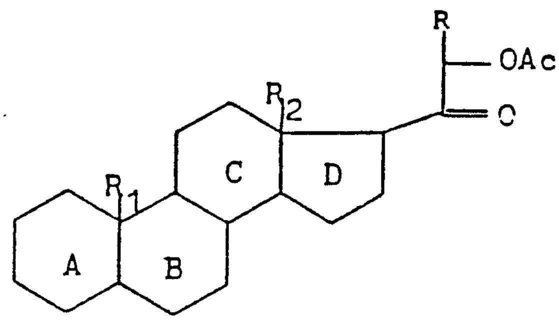

(VI')

L'agent de réduction de la double liaison est de préférence un borohydrure alcalin, comme, par exemple, le borohydrure de sodium; les autres réactifs utilisés de façon préférentielle sont ceux indiqués ci-dessus.

On peut signaler plus particulièrement l'application des composés $I'_A$ et $I''_A$.

On peut mentionner tout spécialement l'application décrite dans la partie expérimentale, caractérisée en ce que le produit de formule $I_A$ utilisé est le 3β-hydroxy 17-(nitrométhylène)androst-5-ène que l'on soumet à l'action d'un agent de formylation, pour obtenir le composé de formule:

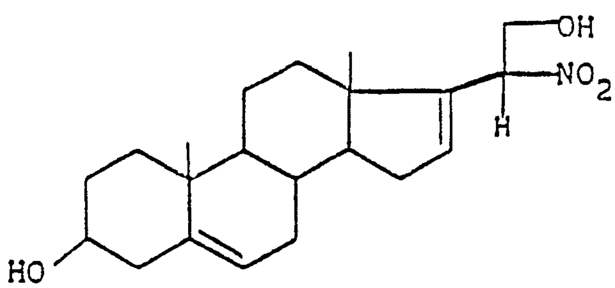

que l'on soumet à l'action d'un agent d'acétylation, pour obtenir le composé de formule:

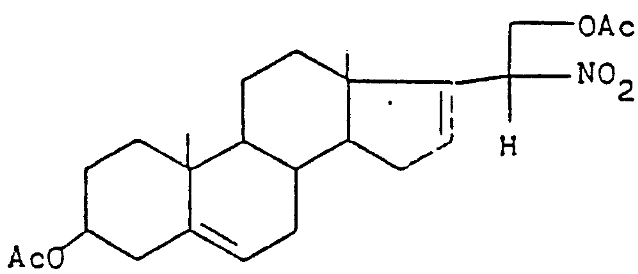

dans laquelle

Ac représente le radical acétyle, que l'on soumet à l'action d'un agent de réduction du groupement nitro pour obtenir le composé de formule:

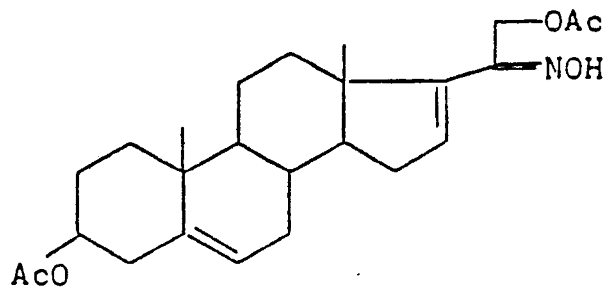

que l'on soumet à l'action d'un agent de clivage de fonction oxime, pour obtenir le composé de formule:

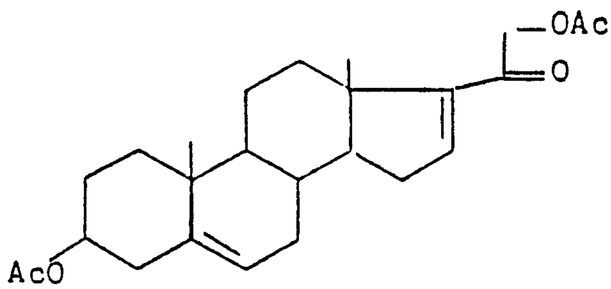

On peut encore indiquer pour objet l'application caractérisée en ce que le produit de formule $I_A$ utilisé est le 3β-hydroxy 17-(nitrométhylène)androst-5-ène que l'on soumet à l'action de réduction pour obtenir le composé de formule:

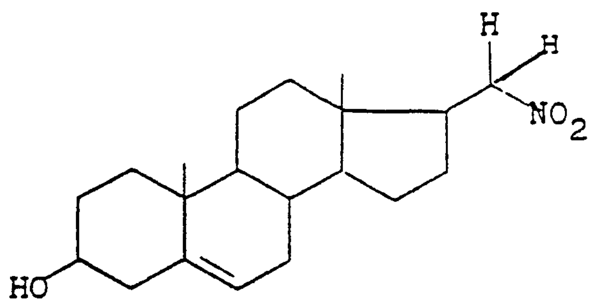

que l'on soumet à l'action d'un agent de formylation, pour obtenir le composé de formule:

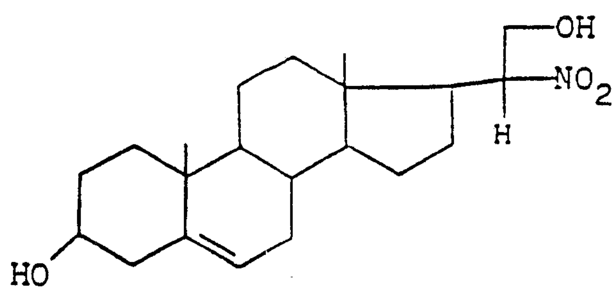

que l'on soumet à l'action d'un agent d'acétylation, pour obtenir le composé de formule:

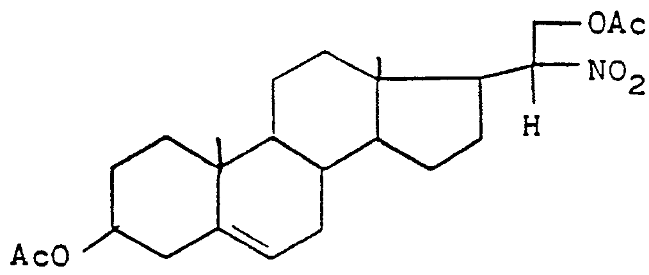

dans laquelle

Ac représente un radical acétyle, que l'on soumet à l'action d'un agent de réduction du groupement nitro pour obtenir le composé de formule:

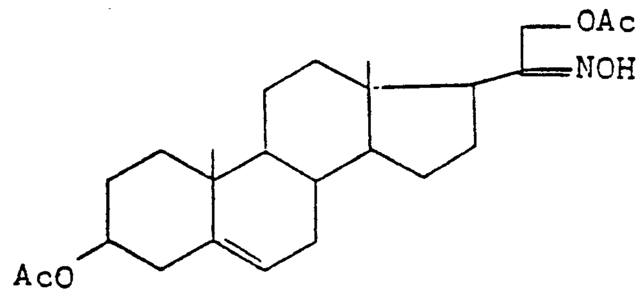

que l'on soumet à un agent de clivage de la fonction oxime, pour obtenir le composé de formule:

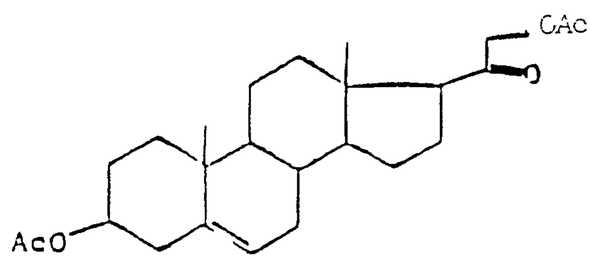

L'invention a bien entendu également pour objet les applications telles que celles menant aux composés de formules (VI) et (VI'), qui ont été définies précédemment, caractérisées en ce que l'on utilise au départ les produits de formule I dans laquelle X représente un atome de carbone.

Les composés de formule III, IV, V, VII, III', IV' et V' obtenus lors de l'application qui vient d'être décrite, ainsi que lors de la variante sont des produits chimiques nouveaux.

Les exemples suivants illustrent l'invention.

**Préparation 1: 3β-hydroxy 17-(nitrométhylène) androst 5-ène**

On chauffe au reflux sous atmosphère d'azote, une solution renfermant 8,7 g de 3β-hydroxy androst-5-ène 17-one dans 150 $cm^3$ de nitrométhane, pour distiller 10 $cm^3$ de nitrométhane. On ajoute 0,1 $cm^3$ d'éthylènediamine et on chauffe le mélange au reflux pendant 50 - 60 heures. On chasse le solvant sous pression réduite, reprend le résidu dans le dichlorométhane et le filtre sur silice en éluant par le mélange éther-dichlorométhane (1/1). On chasse le solvant organique sous pression réduite. On obtient ainsi 9,9 g du produit recherché fondant à 118 - 122°C. Le produit cristallise avec une demie-mole de méthanol. F = 165 - 168°C.

$(\alpha)_D$ = -88° (c = 0,76 % méthanol).

<u>RMN</u> $CCl_4$ ppm

6,65 1H large 20 H

5,25 1H large 6 H

1,05 3H singulet 10 $CH_3$

0,95 3H singulet 13 $CH_3$.

Ce produit est décrit dans la demande de brevet européen 0 087 359.

**Exemple 2**: <u>3,4-dihydro 2-nitrométhane naphtalène.</u>

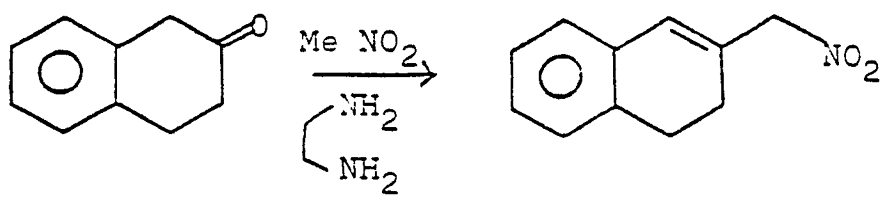


Une solution de 220 mg de β-tétralone dans 3 $cm^3$ de nitrométhane contenant environ 15 mg d'éthylène diamine, est chauffée à 75°C sous azote pendant 18 heures. L'excès de nitrométhane est évaporé sous pression réduite et le résidu est chromatographié sur silice (benzène-pentane 2/3). On obtient 232 mg de produit brut que l'on recristallise dans le pentane, F = 29 - 31°C.

Spectre IR: max 1550 $cm^{-1}$,

RMN $CDCl_3$ ppm
6,9 (4H, aromatiques)
6,4 (1H, singulet large, H éthylénique)
4,9 (2H, singulet $-CH_2-N_2$)
2,3 - 3,0 (4H, m, $-CH_2$).

Analyse

| | | | |
|---|---|---|---|
| Trouvé: | C % 69,96 | H % 5,88 | N % 7,49 |
| Calculé: | 69,83 | 5,86 | 7,40 |

**Exemple 1**: 3β, 21-diacétoxy-prégna 5,16-dièn 20-one.

Stade A: 3β,21-dihydroxy 20-nitro prégna 5,16-diène et diacétate correspondant.

On maintient sous agitation à la température ambiante pendant 30 minutes, un mélange renfermant une suspension de 500 mg de 3β-hydroxy 17-(nitrométhylène) androst 5-ène dans 10 $cm^3$ d'isopropanol, 2 $cm^3$ d'une solution aqueuse de formaldéhyde et 1 $cm^3$ de triéthylamine. On verse la mélange réactionnel dans une solution aqueuse d'acide acétique. On agite pendant 30 minutes, filtre et sèche le produit obtenu. On obtient ainsi 511 mg de produit recherché sous forme de diacétate qui fond à 155 - 163°C.

$(\alpha)_D$ = -52° (c = 0,48 % $CHCl_3$)

RMN $CCl_4$ ppm
5,95: 1H large 16H
5,30: 1H large 6H
4,9 - 5,1: 1H large 20H
4,1 - 4,6: 3H large 3H et 21H
2,05 et 1,97: 6H, singulet acétate
1,10: 3H, singulet $10-CH_3$
0,85: 3H, singulet $13-CH_3$

Stade B: 20-oxime de 3β,21-diacétoxy prégna 5,16-dièn-20-one.

On ajoute 6 $cm^3$ d'une solution d'acide chlorhydrique concentré et 1,8 g de poudre de zinc à une solution renfermant 3 g de $CrCl_3-6H_2O$ dans 14 $cm^3$ d'eau. On filtre la solution obtenue et la verse dans une solution renfermant 1 g de 3β,21-diacétoxy 20-nitro prégna 5,16-diène dans 150 $cm^3$ d'acétone. Après 6 heures d'agitation, on verse la solution obtenue dans une solution aqueuse de chlorure de sodium et extrait à l'éther. On lave la phase éthérée à l'eau salée et la sèche. On filtre sur silice, évapore à sec et obtient 830 mg d'un solide blanc cristallisé fondant à 173 - 176°C.

$(\alpha)_D$ = -42° (c = 1,06 % $CHCl_3$)

RMN $CCl_4$ ppm
5,97 1H large 16 H
5,27 1H large 6 H
4,88 2H, singulet $21-CH_2$
4,3 - 4,7 1H large 3H
2,04 et 2,00 6H, singulet acétate
1,05 3H, singulet $10-CH_3$
0,94 3H, singulet $13-CH_3$

Stade C: 3β,21-diacétoxy prégna 5,16-dièn 20-one.

On ajoute 6,5 $cm^3$ d'une solution aqueuse de trichlorure de titane dans une suspension renfermant 800 mg de 20-oxime de 3β,21-diacétoxy prégna 5,16-dièn-20-one, 16 $cm^3$ d'acide acétique et 6 $cm^3$ d'acétone contenant 2,4

g d'acétate d'ammonium. On maintient le mélange réactionnel sous agitation pendant 6 heures, le verse dans l'eau, et extrait à l'éther. On lave la phase éthérée et la sèche. On filtre sur silice. On obtient après évaporation 766 mg d'un solide blanc fondant à 154 - 156°C.

$(\alpha)_D$ = -39° (c = 0,95 % $CHCl_3$)

**Exemple 2**: Préparation de la 3β,21-diacétoxy pregn-5-en 20-one.

1) 17β-nitrométhyl 3β-hydroxy androst-5-ène.

A une suspension de 0,5 g de 17-nitrométhylène 3β-hydroxy androst-5-ène dans 15 $cm^3$ d'isopropanol, on ajoute, peu à peu en 10 minutes, environ 100 mg de borohydrure de sodium. On agite le mélange à température ambiante pendant 2 heures, verse dans 300 $cm^3$ d'une solution aqueuse d'acide acétique à 1 %. On filtre, sèche et obtient 0,5 g de produit attendu,

F = 178 - 181°C (méthanol).
$(\alpha)_D$ = -70° (c = 1 % $CHCl_3$)
Spectre IR: max 1540 $cm^{-1}$
RMN $CDCl_3$ ppm
5,25 (1H, m, 6-H)
4,25 (2H, m, 20-$CH_2$)
3,4 (1H, large, 3α-H)
1,05 (3H, singulet, 19-$CH_3$)
0,7 (3H, singulet, 18-$CH_3$).

2) 3β,21-diacétoxy 20-nitro-pregn-5-ène.

A une suspension de 1 g de 17β-nitrométhyl 3β-hydroxy androst-5-ène dans 20 $cm^3$ d'isopropanol, on ajoute 4 $cm^3$ de formol et 2 $cm^3$ de triéthylamine. On agite à température ambiante pendant 40 - 50 minutes puis verse dans 200 $cm^3$ d'une solution aqueuse d'acide acétique à 2,5 %. On filtre, sèche et obtient 1,07 g de produit attendu.

F = 187 - 195° C (méthanol).
$(\alpha)_D$ = -22° (c = 0,4 % $CHCl_3$)

3) 3β,21-diacétoxy-prégn-5-èn-20-one.

A une solution de 200 mg de 3β,21-diacétoxy 20-nitro-pregn-5-ène dans 35 $cm^3$ d'acétone on ajoute sous azote une solution filtrée de chlorure chromeux (préparée à partir de 0,6 g de $CrCl_3$-6 $H_2O$ et de 0,3 g de zinc en poudre dans une solution aqueuse d'acide chlorhydrique renfermant 1,5 $cm^3$ d'acide concentré et 2,5 $cm^3$ d'eau). Après 2 - 3 minutes, on verse le mélange dans l'eau et extrait avec du dichlorométhane. La phase organique est séchée et évaporée et le résidu est dissous dans 4 $cm^3$ d'acide acétique. On ajoute goutte à goutte 2,5 $cm^3$ d'une solution aqueuse de nitrite de sodium, puis 4 $cm^3$ d'acide acétique. Le mélange est bouilli pendant 10 minutes, versé dans l'eau, neutralisé par le carbonate de sodium et extrait avec le dichlorométhane. La phase organique est séchée, filtrée sur silice et évaporée pour donner 149 mg de produit attendu.

F = 162 - 164°C
$(\alpha)_D$ = +22° (c = 0,7 % $CHCl_3$)
(Lit. (1) F = 166 - 168°C; $(\alpha)_D$ = +27°)
(1) 1. Marquet et J. Jacques, Bull Soc. Chim. Fr. 1 962,90.

## Revendications

1. A titre de composés nouveaux, les composés de formule $I_A$:

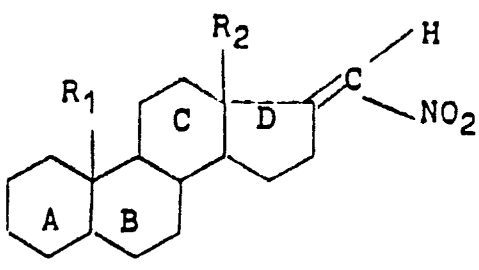

$(I_A)$

dans laquelle $R_1$ représente un atome d'hydrogène, un radical alkyle renfermant de 1 à 4 atomes de carbone éventuellement substitué par une fonction oxygénée ou azotée ou par un atome d'halogène ou $R_1$ représente un radical alkényle ou alkynyle renfermant de 2 à 4 atomes de carbone, $R_2$ représente un radical alkyle renferment de 1 à 4 atomes de carbone, les noyaux A, B, C et D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone ou par un ou plusieurs radicaux alkényle et alkynyle renfermant de 2 à 4 atomes de carbone.

2. A titre de composés tels que définis à la revendication 1, les composés de formule $I_A$, dans lesquels $R_2$ représente un radical méthyle et $R_1$ représente un atome d'hydrogène ou un radical méthyle.

3. A titre de composés tels que définis à la revendication 1, les composés répondant à la formule $I'_A$:

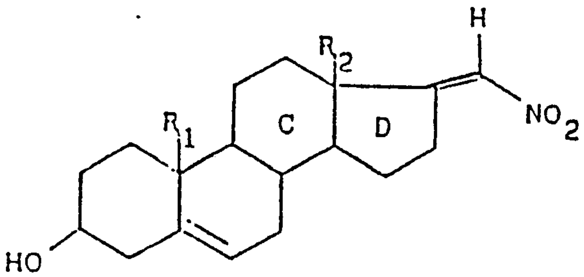

$(I'_A)$

dans laquelle R, $R_2$ C et D conservant leur signification précédente.

4. A titre de composés tels que définis à la revendication 1, les composés de formule $I''_A$:

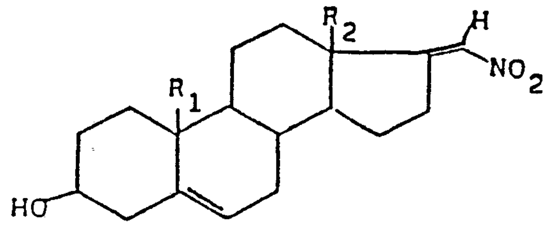

$(I''_A)$

5. A titre de composés définis à la revendication 4, le 3β-hydroxy 17-(nitrométhyl-ène) androst 5-ène.

6. Application des composés de formule $I_A$ tels que définis à la revendication 1, caractérisée en ce que l'on soumet un composé de formule $I_A$ à l'action d'un aldéhyde RCHO dans lequel R représente un atome d'hydrogène ou un radical alkyle renfermant de 1 à 18 atomes de carbone, pour obtenir le composé de formule (III):

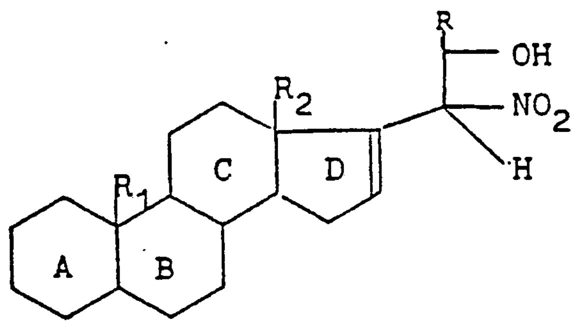

(III)

dans laquelle A, B, C, D, R, $R_1$ et $R_2$ sont définis comme précédemment, que l'on soumet à l'action d'un agent d'estérification, pour obtenir le composé de formule (IV):

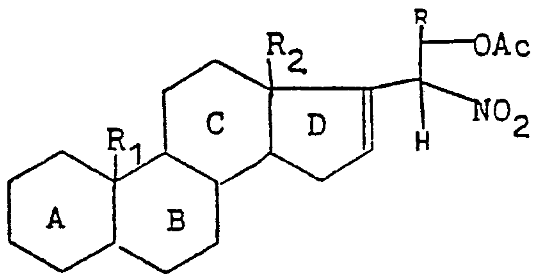

(IV)

dans laquelle Ac représente le reste d'un radical acyle renfermant de 1 à 18 atomes de carbone, que l'on soumet à l'action d'un agent de réduction du groupement nitro, pour obtenir le composé de formule (V):

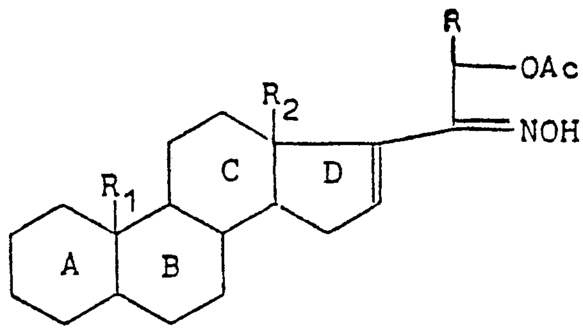

(V)

que l'on soumet à un agent de clivage de la fonction oxime, pour obtenir le composé de formule (VI):

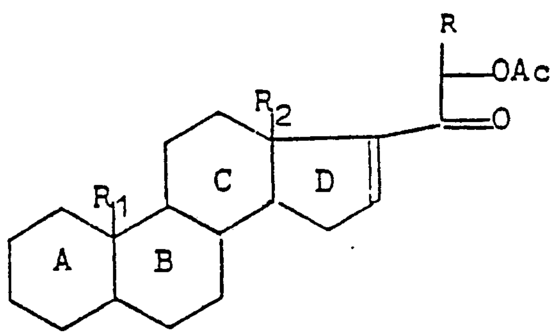

(VI)

7. Application selon la revendication 6, caractérisée en ce que l'on soumet un composé de formule ($I_A$):

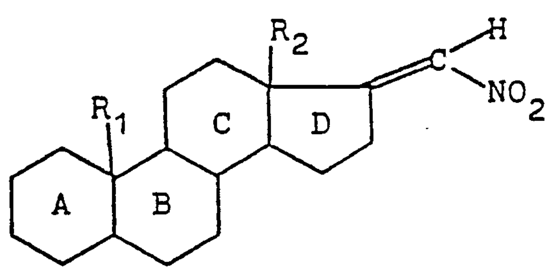

($I_A$)

dans laquelle A, B, C, D, $R_1$ et $R_2$ sont définis comme dans la revendication 5, d'abord à l'action d'un agent de réduction de la double liaison 17(20), pour obtenir le composé de formule (VII):

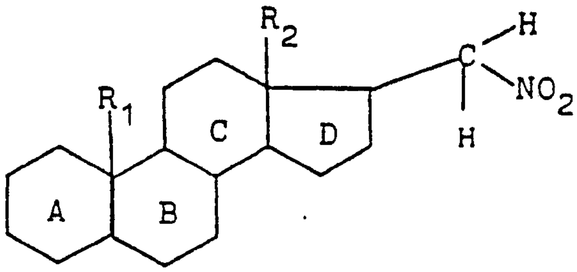

(VII)

que l'on soumet ensuite à la suite des réactions indiquées à la revendication 6, c'est-à-dire à l'action d'un aldéhyde RCHO, dans lequel R est défini comme dans la revendication 6, pour obtenir le composé de formule (III'):

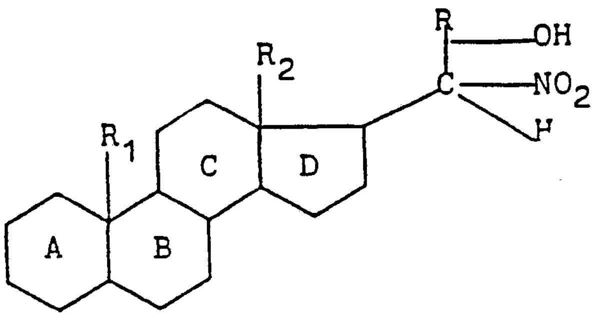

(III')

dans laquelle A, B, C, D, R, $R_1$ et $R_2$ sont définis comme précédemment, que l'on soumet à l'action d'un agent d'estérification, pour obtenir le composé de formule (IV'):

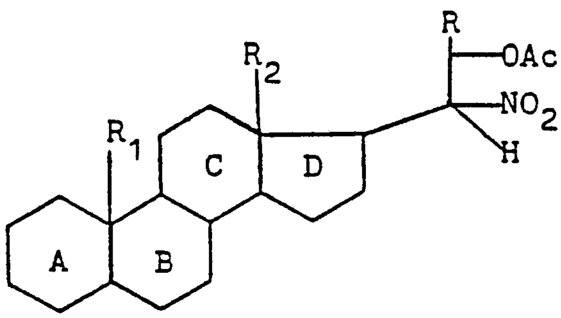

(IV')

dans laquelle Ac représente le reste d'un radical acyle renfermant de 1 à 18 atomes de carbone, que l'on soumet à l'action d'un agent de réduction du groupement nitro, pour obtenir le composé de formule (V'):

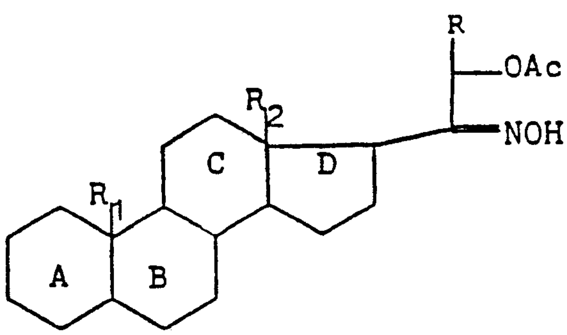

(V')

que l'on soumet à l'action d'un agent de clivage de la fonction oxime, pour obtenir le composé de formule (VI') correspondant:

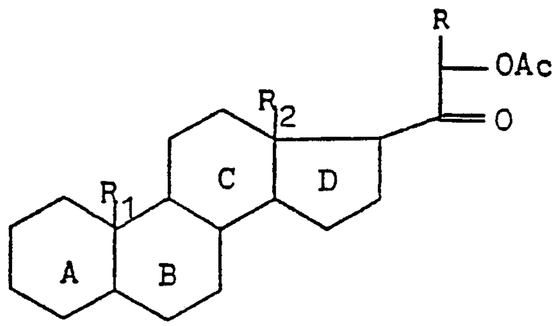

(VI')

8. Application selon la revendication 6, caractérisée en ce que le produit de formule $I_A$ utilisé est le 3β-hydroxy 17-(nitrométhylène)androst-5-ène que l'on soumet à l'action d'un agent de formylation, pour obtenir le composé de formule:

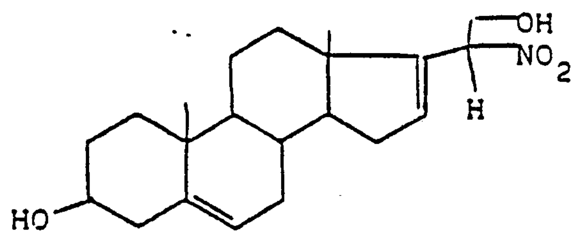

que l'on soumet à l'action d'un agent d'acétylation, pour obtenir le composé de formule:

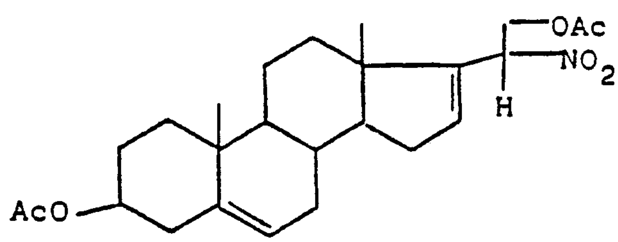

dans laquelle Ac représente le radical acétyle que l'on soumet à l'action d'un agent de réduction du groupement nitro, pour obtenir le composé de formule:

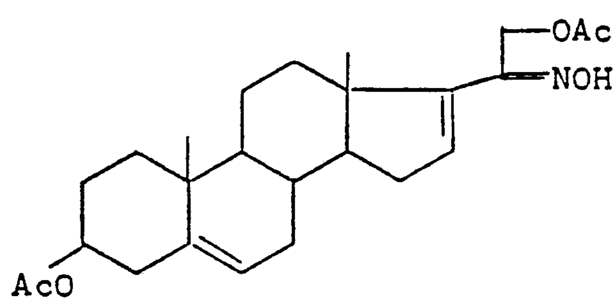

que l'on soumet à l'action d'un agent de clivage de la fonction oxime pour obtenir le composé de formule:

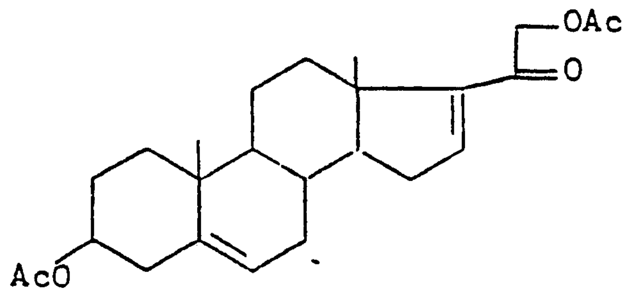

9. Application selon la revendication 7, caractérisée en ce que le produit de formule $I_A$ utilisé est le 3β-hydroxy (17-nitrométhylène)androst-5-ène que l'on soumet à l'action de réduction, pour obtenir le composé de formule:

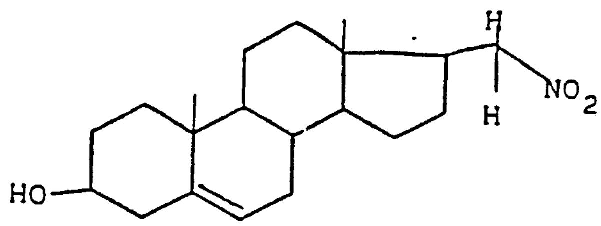

que l'on soumet à l'action d'un agent de formylation, pour obtenir le composé de formule:

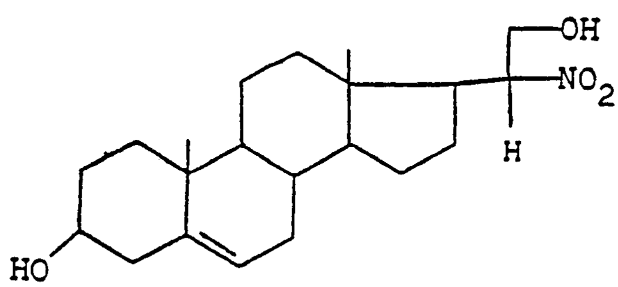

que l'on soumet à l'action d'un agent d'acétylation, pour obtenir le composé de formule:

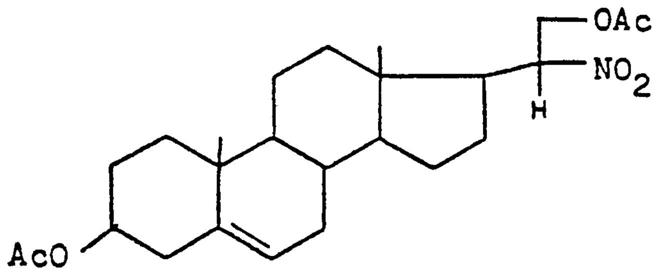

dans laquelle Ac représente un radical acétyle, que l'on soumet à l'action d'un agent de réduction du groupement nitro, pour obtenir le composé de formule:

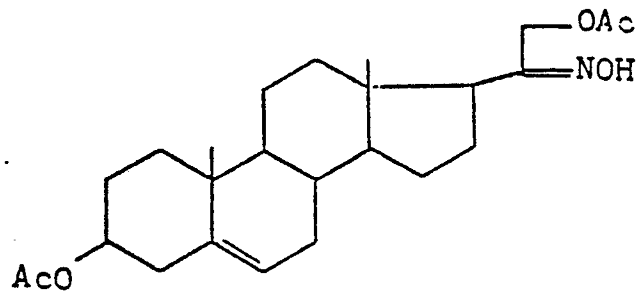

que l'on soumet à un agent de clivage de la fonction oxime, pour obtenir le composé de formule:

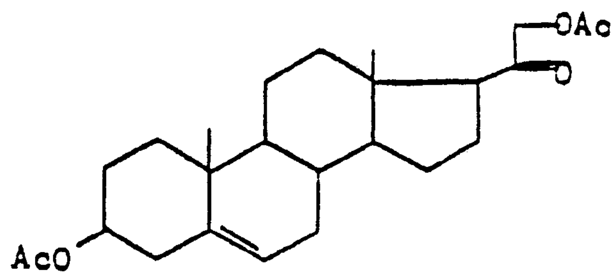

10. A titre de produits chimiques nouveaux, les produits de formules III, IV, V, VII, III', IV' et V' et tels que définis dans les revendications 6 et 7.

**Patentansprüche**

1. Als neue Verbindungen die Verbindungen der Formel $I_A$:

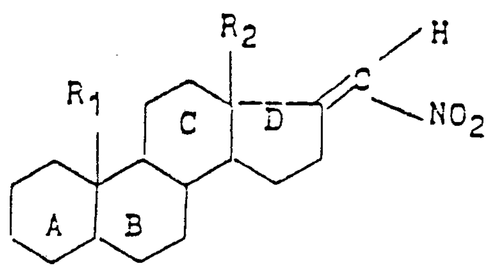

$(I_A)$

worin $R_1$ für ein Wasserstoffatom, einen Alkylrest mit 1 bis 4 Kohlenstoffatomen, gegebenenfalls substituiert durch eine Sauerstoff- oder Stickstoffunktion oder durch ein Halogenatom, steht oder $R_1$ eine Alkenyl- oder Alkinylgruppe mit 2 bis 4 Kohlenstoffatomen wiedergibt, $R_2$ einen Alkylrest mit 1 bis 4 Kohlenstoffatomen bedeutet, die Ringe A, B, C und D gegebenenfalls eine oder mehrere Doppelbindungen aufweisen und gegebenenfalls durch eine oder mehrere Hydroxyl- oder Ketofunktionen, durch ein oder mehrere Halogenatome, durch einen oder mehrere Alkyl- oder Alkoxyreste mit 1 bis 4 Kohlenstoffatomen oder durch einen oder mehrere Alkenyl- oder Alkinylreste mit 2 bis 4 Kohlenstoffatomen substituiert sind.

2. Als Verbindungen gemäß Anspruch 1 die Verbindungen der Formel $I_A$, worin $R_2$ für einen Methylrest steht und $R_1$ ein Wasserstoffatom oder einen Methylrest bedeutet.

3. Als Verbindungen gemäß Anspruch 1 die Verbindungen der Formel $I'_A$:

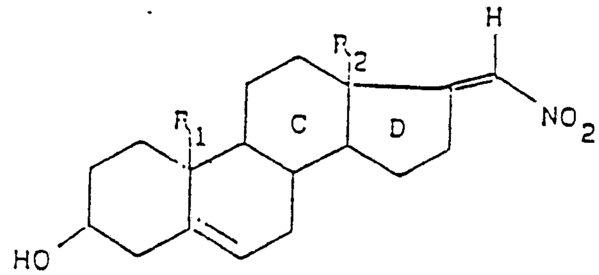

($I'_A$)

worin R, $R_2$, C und D die angegebene Bedeutung besitzen.

4. Als Verbindungen gemäß Anspruch 1 die Verbindungen der Formel $I''_A$:

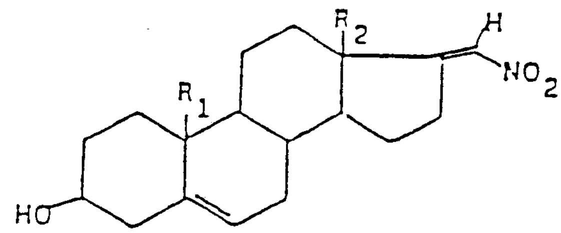

($I''_A$)

5. Als Verbindungen gemäß Anspruch 4 das 3-Hydroxy-17-(nitromethylen)-androst-5-en.

6. Verwendung der Verbindungen der Formel $I_A$ gemäß Anspruch 1, dadurch gekennzeichnet, daß man eine Verbindung der Formel $I_A$ der Einwirkung eines Aldehyds RCHO unterzieht, worin R für ein Wasserstoffatom oder einen Alkylrest mit 1 bis 18 Kohlenstoffatomen steht, um zu der Verbindung der Formel (III):

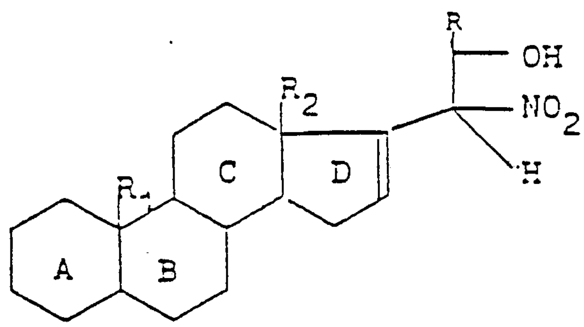

(III)

zu gelangen, worin A, B, C, D, R, $R_1$ und $R_2$ wie vorstehend definiert sind, die man der Einwirkung eines Veresterungsmittels unterzieht, um zu der Verbindung der Formel (IV):

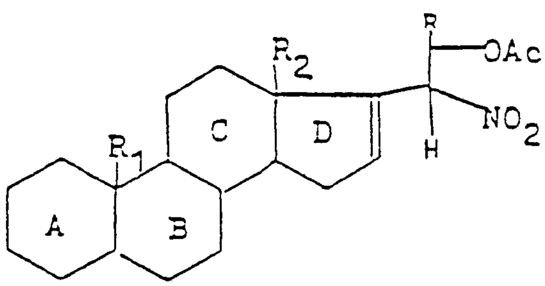

(IV)

zu gelangen, worin Ac den Rest eines Acylrestes mit 1 bis 18 Kohlenstoffatomen bedeutet, welche man der Einwirkung eines Reduktionsmittels für die Nitrogruppe unterzieht, um die Verbindung der Formel (V):

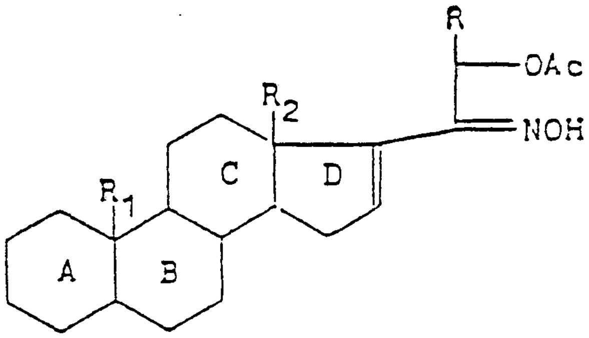

(V)

zu erhalten, die man der Einwirkung eines Mittels zur Abspaltung der Oximfunktion unterzieht, um die Verbindung der Formel (VI):

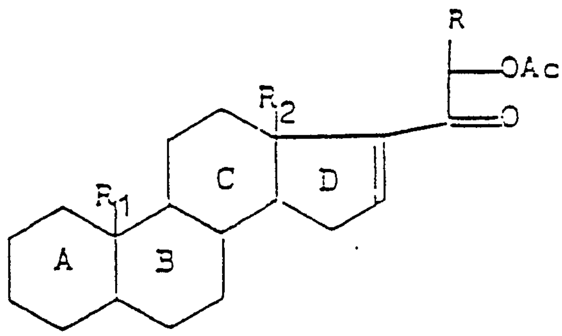

(VI)

zu erhalten.

7. Verwendung gemäß Anspruch 6, dadurch gekennzeichnet, daß man eine Verbindung der Formel ($I_A$):

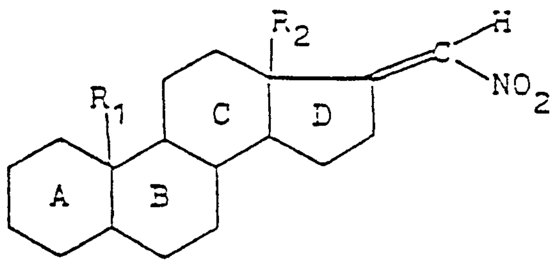

($I_A$)

worin A, B, C, D, $R_1$ und $R_2$ wie in Anspruch 5 definiert sind, zunächst der Einwirkung eines Mittels zur Reduktion der 17(20)-Doppelbindung unterzieht, um zu der Verbindung der Formel (VII):

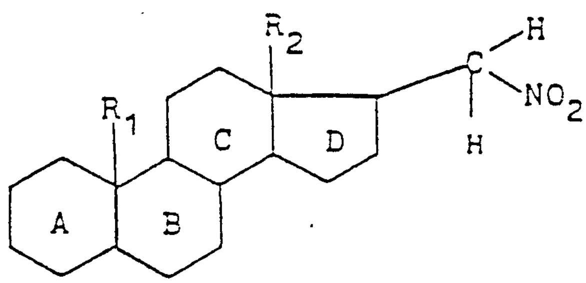

(VII)

zu gelangen, die man hierauf der in Anspruch 6 angegebenen Reaktionsfolge unterzieht, d.h. der Einwirkung eines Aldehyds RCHO, worin R wie in Anspruch 6 definiert ist, um zu der Verbindung der Formel (III'):

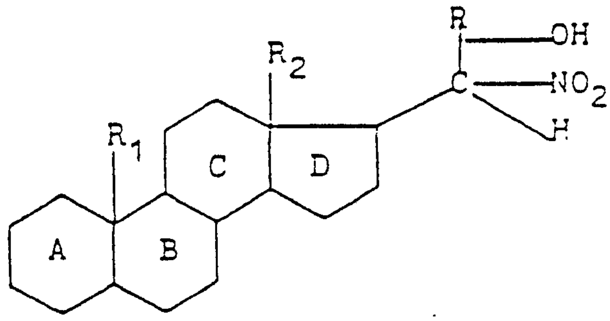

(III')

zu gelangen, worin A, B, C, D, R, $R_1$ und $R_2$ wie vorstehend definiert sind, die man der Einwirkung eines Veresterungsmittels unterzieht, um zu der Verbindung der Formel (IV'):

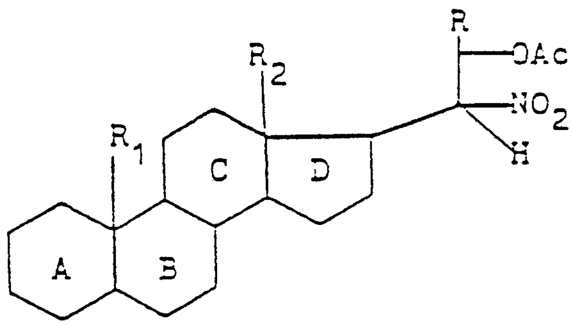

(IV')

zu gelangen, worin Ac den Rest eines Acylrestes mit 1 bis 18 Kohlenstoffatomen wiedergibt, die man der Einwirkung eines Reduktionsmittels für die Nitrogruppe unterzieht, um zu der Verbindung der Formel (V'):

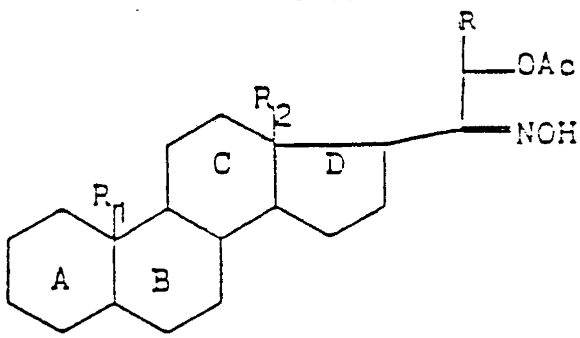

(V')

zu gelangen, die man der Einwirkung eines Mittels zur Abspaltung der Oximfunktion unterzieht, um zu der entsprechenden Verbindung der Formel (VI'):

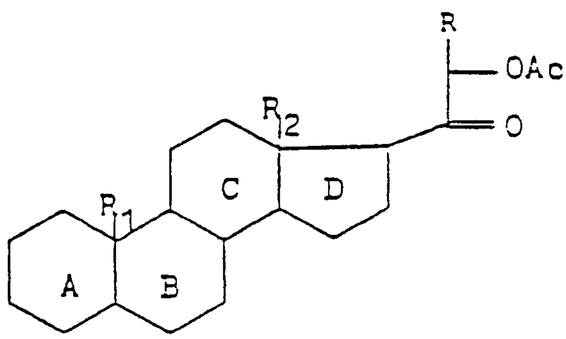

(VI')

zu gelangen.

8. Verwendung gemäß Anspruch 6, dadurch gekennzeichnet, daß das verwendete Produkt der Formel $I_A$ das 3β-Hydroxy-17-(nitromethylen)-androst-5-en ist, welches man der Einwirkung eines Formylierungsmittels unterzieht, um zu der Verbindung der Formel:

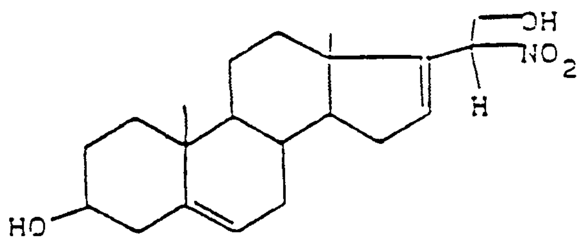

zu gelangen die man der Einwirkung eines Acetylierungsmittels unterzieht, um zu der Verbindung der Formel:

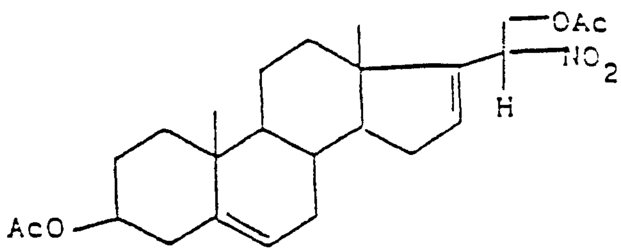

zu gelangen, worin Ac den Acetylrest bedeutet, die man der Einwirkung eines Reduktionsmittels für die Nitrogruppe unterzieht, um die Verbindung der Formel:

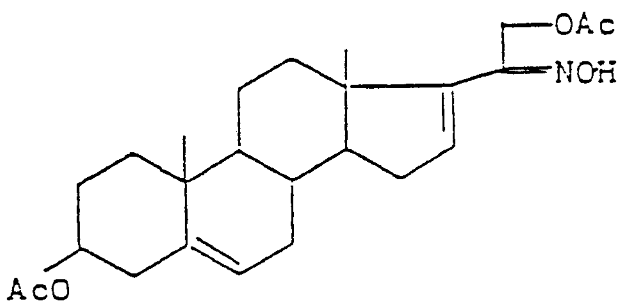

zu erhalten, die man der Einwirkung eines Mitteis zur Abspaltung der Oximfunktion unterzieht, um die Verbindung der Formel:

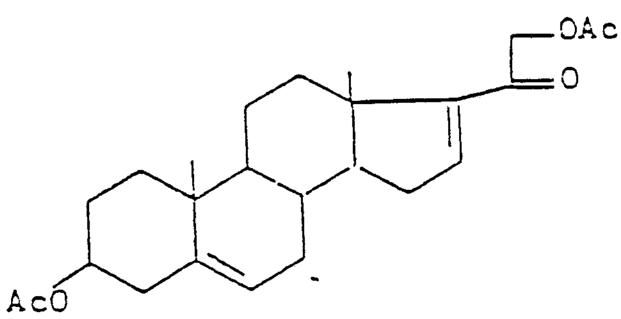

zu erhalten.

9. Verwendung gemäß Anspruch 7, dadurch gekennzeichnet, daß das verwendete Produkt der Formel $I_A$ das 3β-Hydroxy-(17-nitromethylen)-androst-5-en ist, welches man einer Reduktion unterzieht, um die Verbindung der Formel:

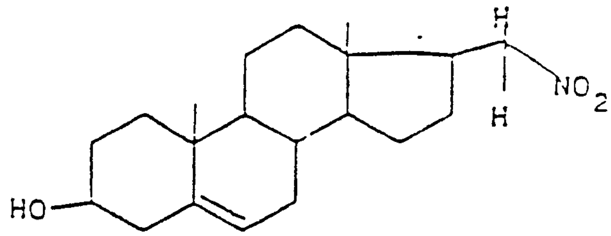

zu erhalten, die man der Einwirkung eines Formylierungsmittels unterzieht, um die Verbindung der Formel:

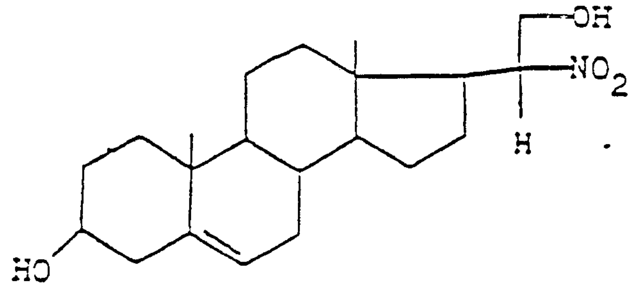

zu erhalten, die man der Einwirkung eines Acetylierungsmittels unterzieht, um die Verbindung der Formel:

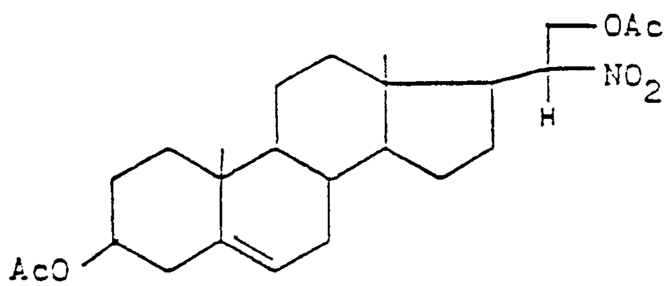

zu erhalten, worin Ac für einen Acetylrest steht, die man der Einwirkung eines Reduktionsmittels für die Nitrogruppe unterzieht, um die Verbindung der Formel:

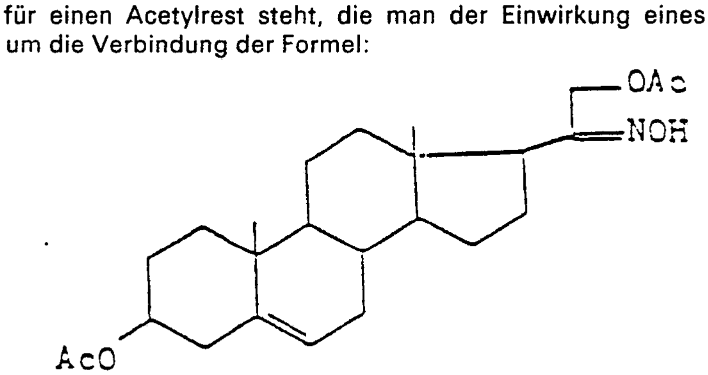

zu erhalten, die man der Einwirkung eines Mittels zur Abspaltung der Oximfunktion unterzieht, um die Verbindung der Formel:

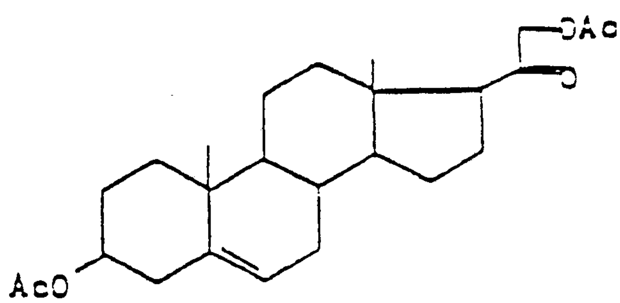

zu erhalten.

10. Als neue, chemische Produkte die Produkte der Formeln III, IV, V, VII, III', IV' und V' und die gemäß den Ansprüchen 6 und 7 definierten.

## Claims

1. As new compounds, compounds of formula $I_A$:

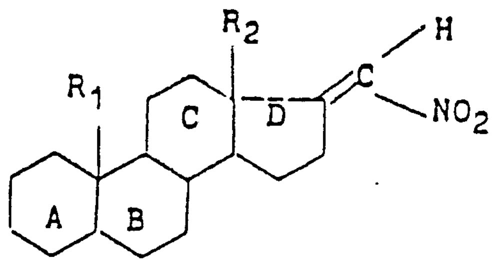

$(I_A)$

in which $R_1$ represents a hydrogen atom, an alkyl radical containing from 1 to 4 carbon atoms optionally substituted by an oxygenated or nitrogenized function or by a halogen atom or $R_1$ represents an alkenyl or alkynyl radical containing from 2 to 4 carbon atoms, $R_2$ represents an alkyl radical containing from 1 to 4 carton atoms, the nuclei A, B, C and D optionally carry one or more double bonds and are optionally substituted by one or more hydroxyl or ketone functions, by one or more halogen atoms, by one or more alkyl or alkoxy radicals containing from 1 to 4 carton atoms or by one or more alkenyl or alkynyl radicals containing from 2 to 4 carbon atoms.

2. As compounds as defined in claim 1, compounds of formula $I_A$, in which $R_2$ represents a methyl radical and $R_1$ represents a hydrogen atom or a methyl radical.

3. As compounds as defined in claim 1, compounds corresponding to the formula $I'_A$:

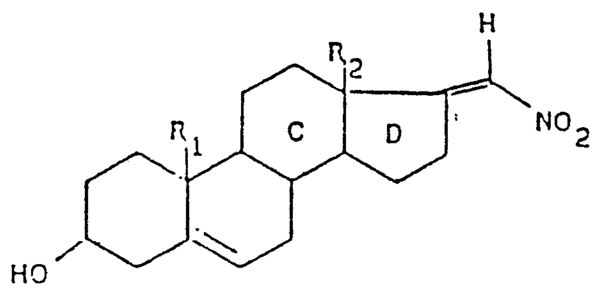

$(I'_A)$

in which R, $R_2$, C and D keep their previous meaning.

4. As compounds as defined in claim 1, compounds of formula $I''_A$:

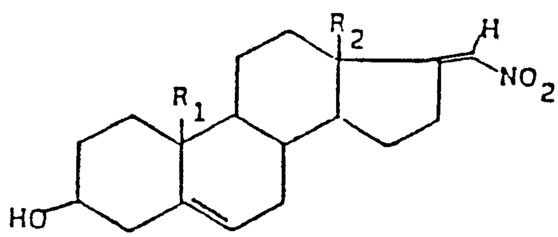

$(I''_A)$

5. As compounds defined in claim 5, 3beta-hydroxy 17-(nitromethyl-ene) androst-5-ene.

6. Use of the compounds of formula $I_A$ as defined in claim 1, characterized in that a compound of formula $I_A$ is submitted to the action of an RCHO aldehyde in which R represents a hydrogen atom or an alkyl radical containing from 1 to 18 carton atoms, to obtain the compound of formula (III):

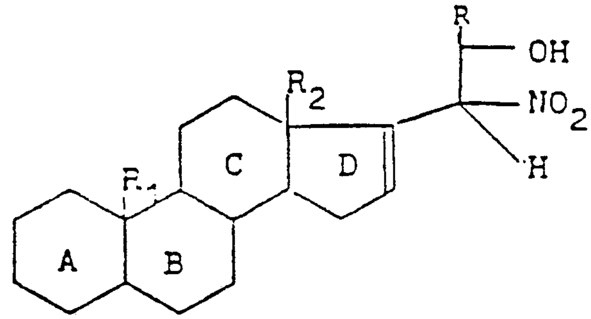

(III)

in which A, B, C, D, R, $R_1$ and $R_2$ are defined as above, which compound is submitted to an esterification agent to obtain the compound of formula (IV):

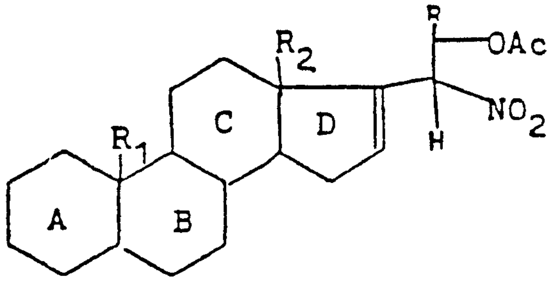

(IV)

in which Ac represents an acyl radical residue containing from 1 to 18 carbon atoms, which is submitted to the action of a reduction agent of the nitro group, to obtain the compound of formula (V):

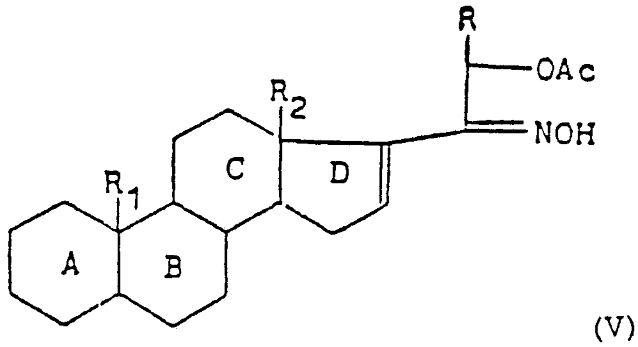

(V)

which compound is submitted to a cleavage agent of the oxime function to obtain the compound of formula (VI):

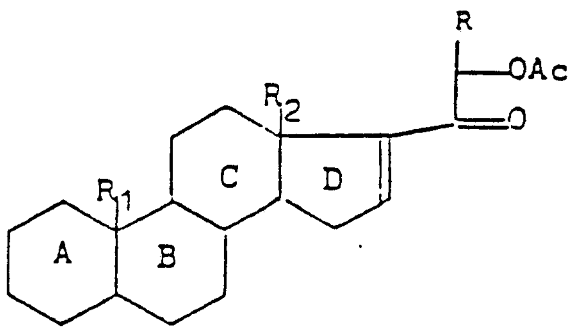

(VI)

7. Use according to claim 6, characterized in that a compound of formula ($I_A$):

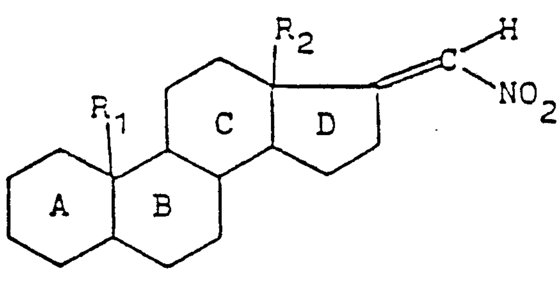

($I_A$)

in which A, B, C, D, $R_1$ and $R_2$ are defined as in claim 5, is first submitted to the action of a reduction agent of the double bond 17(20) to obtain the compound of formula (VII):

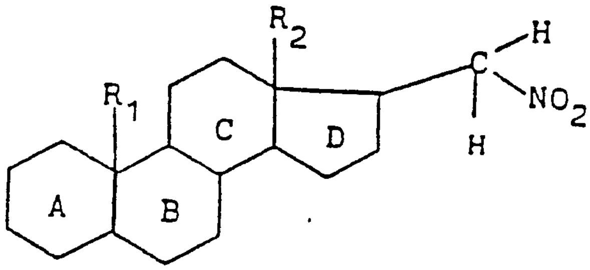

(VII)

which compound is then submitted to the series of reactions, indicated in claim 6, that is to say to the action of an RCHO aldehyde, in which R is defined as in claim 6, to obtain the compound of formula (III'):

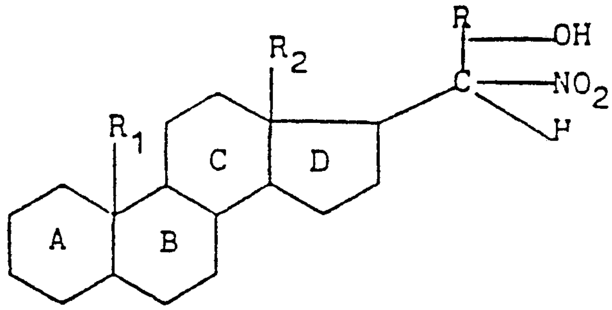

(III')

in which A, B, C, D, R, $R_1$ and $R_2$ are defined as above, which compound is submitted to the action of an esterification agent, to obtain the compound of formula (IV'):

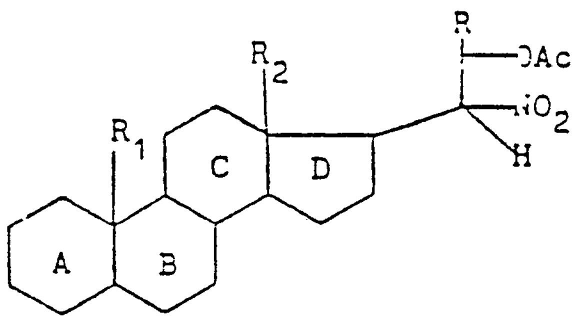

(IV')

in which Ac represents an acyl radical residue containing from 1 to 18 carbon atoms, which compound is submitted to the action of a reduction agent of a nitro group, to obtain the compound of formula (V'):

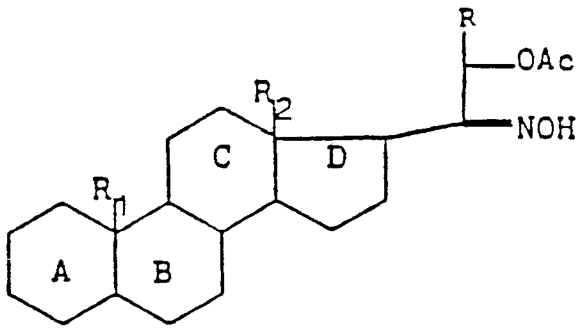

(V')

which is submitted to the action of a cleavage agent of the oxime function to obtain the corresponding compound of formula (VI'):

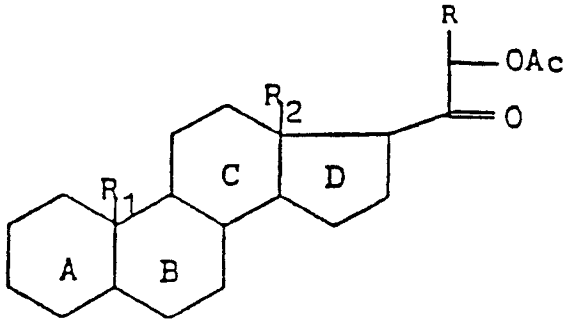

(VI')

8. Use according to claim 6, characterized in that the product of formula $I_A$ used is 3beta-hydroxy 17-(nitromethylene)androst-5-ene which is submitted to the action of a formylation agent to obtain the compound of formula:

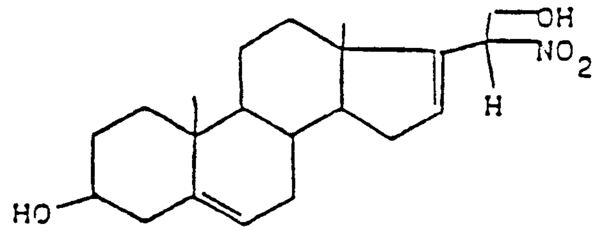

which is submitted to the action of an acetylation agent, to obtain the compound of formula:

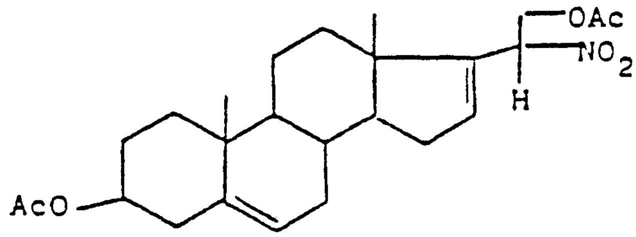

in which Ac represents an acetyl radical, which compound is submitted to the action of a reduction agent of the nitro group to obtain the compound of formula:

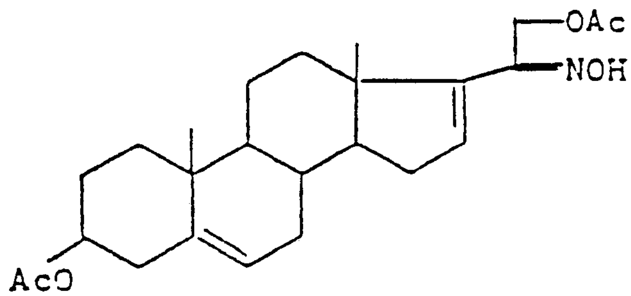

which compound is submitted to the action of a cleavage agent of the oxime function, to obtain the compound of formula:

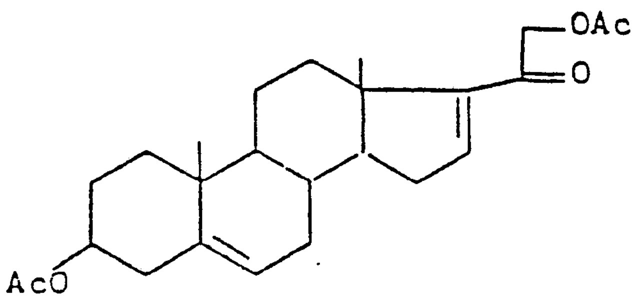

9. Use according to claim 7, characterized in that the product of formula $I_A$ used is 3beta-hydroxy (17-nitromethylene)androst-5-ene which is submitted to the action of a reduction agent to obtain the compound of formula:

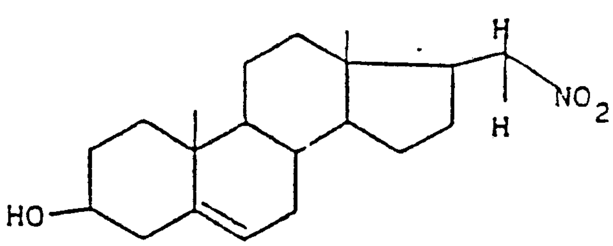

which is submitted to the action of a formylation agent to obtain the compound of formula:

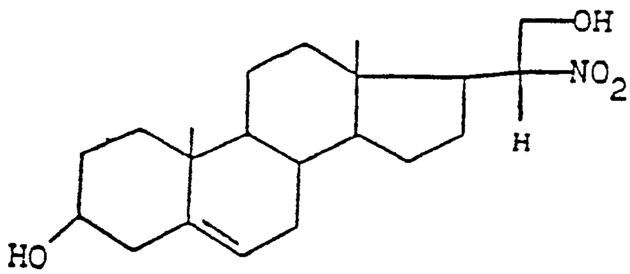

which is submitted to the action of an acetylation agent to obtain the compound of formula:

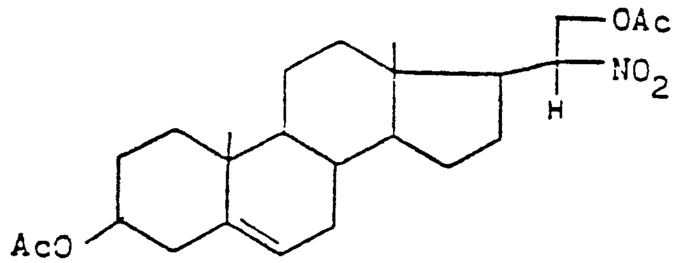

in which Ac represents an acetyl radical, which compound is submitted to the action of a reduction agent of the nitro group to obtain a compound of formula:

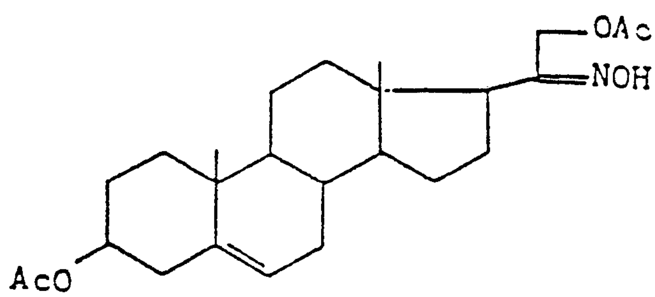

which compound is submitted to the action of a cleavage agent of the oxime function to obtain the compound of formula:

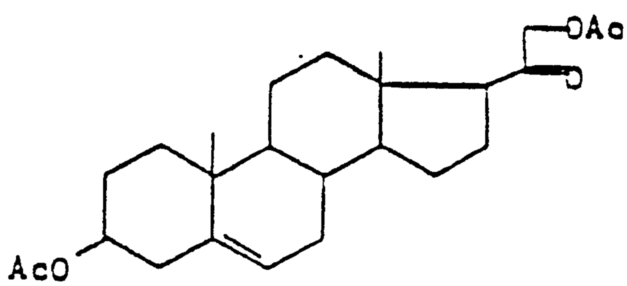

10. As new chemical products, products of formulae III, IV, V, VII, III, IV' and V' and those defined in the claims 6 and 7.